# EUROPEAN PATENT APPLICATION

(11) **EP 4 198 497 A1**
(43) Date of publication of application: **21.06.2023**
(21) Application number: 21214484.4
(22) Date of filing: 14.12.2021
(51) Int. Cl.: G01N 21/3563, G01N 21/25, A47J 31/42, G01N 33/02, A47J 31/52, A47J 42/00, G01J 3/46, A23F 5/00, G01N 21/47, G01N 21/84

(54) **COFFEE BEAN ANALYSIS SYSTEM, COFFEE MACHINE WITH COFFEE BEAN ANALYSIS, AND COFFEE GRINDING SYSTEM WITH COFFEE BEAN ANALYSIS**

(71) Applicant: Versuni Holding B.V., 5656 AE Eindhoven (NL)
(72) Inventor: SINNEMA, Anke Gerda, 5656 AE Eindhoven (NL); SIETZEMA, Ben, 5656 AE Eindhoven (NL)
(74) Representative: Vollering, Stefanus Franciscus Maria

(57) **Abstract**

A system is provided for analyzing coffee beans. A light sensor senses a level of darkness of a coffee bean and a distance sensor is used to determine a distance between the light sensor and the coffee bean. The sensed level of darkness is then calibrated using the determined distance, thereby to obtain a calibrated (and thereby more accurate) level of darkness. This represents a roasting intensity and may be used by a coffee machine to adjust coffee brewing parameters.

## Description

### FIELD OF THE INVENTION

This invention relates to the analysis of coffee beans, for example for enabling automatic control of a coffee or espresso machine.

### BACKGROUND OF THE INVENTION

It is well known that coffee made from freshly ground beans gives better quality than pre-ground coffee. Often, fresh beans are used when making espresso coffee.

There are many different types of commercially available espresso coffee machine, for use in the home, or in bars, restaurants and hotels. The type of machine which is appropriate in a particular setting for example depends on the amount of use, and the budget.

In a manual espresso machine, a user fills a coffee receiving vessel, known as a portafilter, with coffee grounds. The user then needs to tamp the coffee grounds within the portafilter with sufficient pressure, such as around 200N, to create a so-called puck. The portafilter is then mounted to the coffee machine, usually via a bayonet type of connection. Next, the coffee machine drives hot water through the puck in the portafilter and the resulting coffee is dispensed via a spout that is typically integrated in the portafilter. After brewing, the user needs to disconnect and empty the portafilter, throwing away the used coffee grounds.

There are also manual espresso machines with an integrated grinder. A user switches the portafilter between a first position where it receives ground coffee and a second position where coffee is brewed. Tamping may be done manually or via a manually operated lever.

In a fully automatic espresso machine, all of the above-mentioned steps are done automatically, in one and the same machine. The machine comprises a bean reservoir and a grinder, to make the coffee grounds. These grounds are transported into a brew chamber and tamped automatically, via a piston that may be hydraulically actuated or actuated via an electromotor. Next, hot water is driven through the coffee grounds in the brew chamber, coffee is brewed and dispensed, and the used coffee grounds are discharged from the brew chamber into a waste bin within the machine.

This removes the manual steps required by a manual espresso machine and hence saves time, as well as ensures more uniform results.

A manual espresso machine can be produced at lower cost than a fully automatic espresso machine, since many of the transporting steps do not need to be automated. However, the results may be less uniform, as a result of the user involvement in the filling and tamping process, in particular the user involvement in setting the volume or weight of coffee grounds, and the force and uniformity (straightness) of tamping of the coffee grounds into a puck. A manual espresso machine needs less maintenance since the coffee puck is removed after every brew by the consumer as part of the brewing process.

A fully automatic machine gives more consistent results, but is more costly. It also removes the barista feel of using a manual espresso machine. The fully automatic coffee machine also needs more maintenance (filling of coffee beans, water filing, removing waste water and getting rid of the coffee waste in the waste bin). The fully automatic coffee machine may also be bigger.

A third type of coffee machine has been proposed, which combines elements from the two types described above. US 9 125 519 discloses a coffee machine with a removable portafilter as used in a manual espresso machine, but which also includes a bean reservoir and a coffee mill for delivering coffee powder to an inserted portafilter. The portafilter functions as the brewing chamber, and a distribution filter (forming a plunger) is used for automatically tamping and thereby compacting the coffee powder in the portafilter before pressurized hot water is delivered to the portafilter.

The user connects an empty portafilter to the machine by the grinding, dosing of the ground coffee in the portafilter, tamping of the ground coffee, hot water delivery and coffee dispensing is then automated as in a fully automatic machine. After brewing, the user needs to disconnect the portafilter and discharge the coffee waste, similarly to the way a manual espresso machine is used.

Regardless of the type of coffee machine, different coffee brewing processes are suitable for different coffee beans. In particular the roast intensity, which determines the darkness of the coffee beans, influences the coffee brewing parameters that are appropriate.

This invention relates to the analysis of coffee beans to determine a roast intensity, for example to enable selection of suitable brewing parameters, for example so that automatic control of brewing parameters may be implemented. It is of particular interest for a coffee machine which includes a coffee grinder, such as a fully automatic coffee machine.

DE102019200788 discloses a fully automatic coffee machine in which brewing parameters are controlled automatically based on parameters of the coffee beans. One example makes use of a camera to sense a coffee bean color, and this enables a degree of roasting to be determined.

Color determination requires color image sensing, which may not be accurate as it depends heavily on the lighting conditions, and it may be expensive to implement.

There is therefore a need for a simple but accurate way to determine a coffee roasting degree.

### SUMMARY OF THE INVENTION

The invention is defined by the claims.

Examples in accordance with an aspect of the invention provide a system for analyzing coffee beans, comprising:
a light sensor for sensing a level of darkness of a coffee bean;
a distance sensor for determining a distance between the light sensor and the coffee bean; and
a processor adapted to calibrate the sensed level of darkness using the determined distance, thereby to obtain a calibrated level of darkness.

This system measures a level of darkness of a coffee bean, rather than a color, and this enables simpler sensing hardware. To make the darkness determination accurate, the darkness level is calibrated using a distance measurement. Thus, the level of darkness is detected remotely, and the distance to the coffee beans is measured and used to calibrate the darkness measurement so that it is more accurate and can be used to determine accurately a roast level.

The roast level is a continuous scale, but typically roasters divide their beans into three or more categories. Thus, the roast level may be identified as one of three to five categories. The relationship between roast color and roast level is relatively independent of bean type so that a darkness measurement is a good indicator of roast level for all bean types.

It is noted that the light sensor, distance sensor and processor are not necessarily separate components, and indeed as will become clear below, various hardware and software components may be shared to deliver the three functions.

The light sensor is for example for measuring a reflectance from the coffee bean, in particular reflectance of a generated interrogation signal. In this way, the illumination conditions are fixed by the interrogation signal giving repeatable and reliable results.

The distance sensor for example comprises a time of flight sensor. This enables the distance to be measured for different coffee bean positions, for example different coffee bean levels in a coffee reservoir.

The time of flight sensor for example comprises a light source, and the time of flight sensor uses the light sensor to determine a time of flight. Thus, the same light sensor is used for darkness determination as for time of flight measurement.

The light source may comprise an infrared laser and the light sensor is adapted to sense infrared light.

Thus, an infrared sensor is used for darkness determination (based on the level of reflection of the IR light) as well as for a time of flight measurement. Thus, only a few components are needed to implement both darkness sensing and time of flight measurement.

The processor is for example adapted to output a roast level from the calibrated level of darkness. This enables a user to know the bean roast level, and they may make manual adjustments using this information.

The invention also provides a coffee machine, comprising:
a water reservoir;
a water heater;
a water pump;
a coffee bean reservoir;
a coffee grinder having a ground coffee outlet;
a coffee vessel for receiving ground coffee from the coffee grinder;
a water delivery system for delivering heated water to the coffee vessel;
the analyzing system defined above for determining a roast level of coffee beans in the coffee bean reservoir; and
a controller for controlling coffee brewing.

This coffee machine incorporates an analysis of coffee bean roast level into the coffee bean reservoir. This information may be used by the controller in various ways.

For example, the controller may be adapted to control, in dependence on the determined roast level, at least one of:
coffee brewing parameters;
coffee grinding parameters;
the dosing of ground coffee to the coffee vessel.

The controller of the coffee machine may provide an output to a user indicating one or more of:
the determined roast level;
coffee recipe advice for the determined roast level;
coffee taste options for the determined roast level;
a detected change in the coffee bean type.

For example if new beans have been filled into the machine, this can be detected and if the beans are different this can be notified to the user.

The processor of the analyzing system may be further adapted to determine a quantity of coffee beans in the coffee bean reservoir based on the determined distance. Thus, the same analysis system may also be used for determining the fill status of the coffee bean reservoir. This information may be used in various ways by the coffee machine.

For example, the coffee machine controller may be adapted to control, in dependence on the determined quantity of coffee beans, the coffee grinding (grinding time or grinding setting).

The controller may instead or as well be adapted to determine, in dependence on the determined quantity of coffee beans, which coffee recipes can be completed.

Thus, the starting of coffee grinding may be allowed or prohibited depending on whether there are enough beans. This can also depend on the recipe selection, for example a single shot may still be possible, whereas a double shot is not possible. A strong coffee (which requires more coffee beans) may not be possible whereas a mild coffee is possible. Coffee recipes which can be still made with the remaining coffee beans left may be lit up to the user and others may be be faded out.

The controller may also implement automatic bean ordering, in dependence on the determined quantity of coffee. The controller may also provide an output signal indicating the remaining quantity of coffee beans so that a user can also make their own ordering and filling decisions.

The invention also provides a coffee grinding system, comprising:
a coffee bean reservoir for coffee beans;
a coffee grinder;
the analyzing system defined above for determining a roast level of coffee beans in the coffee bean reservoir; and
a controller for controlling coffee the coffee grinder settings based on the roast level.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows the general design of a coffee machine to which the invention may be applied;
Fig. 2 shows an example of a possible hydraulic circuit for a fully automatic coffee machine;
Fig. 3 shows a system for analyzing coffee beans, in particular in a coffee bean reservoir;
Fig. 4 shows the system components;
Fig. 5 is used to show how the calibration enables more accurate darkness determination;
Fig. 6 shows some shape parameters for a coffee bean reservoir; and
Fig. 7 shows the results of a test to demonstrate that an amount of coffee beans can be determined from a distance measurement.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for analyzing coffee beans. A light sensor senses a level of darkness of a coffee bean and a distance sensor is used to determine a distance between the light sensor and the coffee bean. The sensed level of darkness is then calibrated using the determined distance, thereby to obtain a calibrated (and thereby more accurate) level of darkness. This represents a roasting intensity and may be used by a coffee machine to adjust coffee brewing parameters.

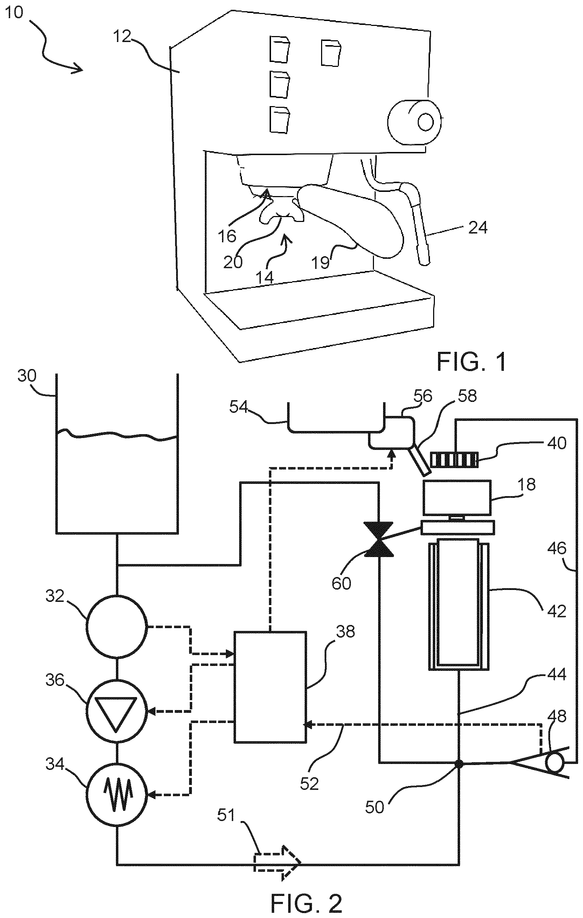

Fig. 1 shows the general design of a coffee machine to which the invention may be applied. Fig. 1 represents a hybrid espresso coffee machine in which coffee grinding takes place internally within the coffee machine. The invention may alternatively be applied to a fully automatic coffee machine.

The coffee machine 10 comprises a main housing 12 having an exterior mounting port 14, for receiving a coffee vessel 16. The illustrated machine further comprises a steam nozzle 24.

The coffee vessel 16 may comprise a filter or filter basket and a support for accommodating the filter or filter basket. The coffee vessel 16 may further comprise a lower spout 20 for dispensing brewed coffee. It may further comprise a handle 19. The coffee vessel 16 may thus correspond to a conventional portafilter, as illustrated in the Figures. In use, the coffee vessel 16 may be fitted to the exterior mounting port 14, for example via a bayonet type coupling.

The coffee vessel forms the brew chamber in use. In the case of a hybrid machine as shown in Figure 1, brew chamber may be considered to be at least partly external, because it is formed by an external, removable part. However, the ground coffee is delivered internally. In the case of a fully automatic machine, the brew chamber is internal.

The main housing 12 contains a hydraulic circuit which provides fluid couplings between a water supply (typically a water reservoir), an internal water heater and a water delivery system having a water delivery head for delivering heated water to the coffee vessel 16.

Fig. 2 shows an example of a possible hydraulic circuit of a fully automatic coffee machine (with with an internal grinder and a coffee brew chamber 18).

Fig. 2 shows a water reservoir 30, a flow meter 32 (for flow rate and dosing control), a water heater 34, a water pump 36 and a controller 38. The flow meter 32 may provide a flow measurement to controller 38, and the controller 38 may control the heater and pump to perform the coffee brewing process. The water heater may for instance be a flow through heater, e.g. a thermoblock. The flow meter 32 is optional. Dosing and flow rate control may alternatively be done via suitable control of the pump 36 (e.g. power level and pump time).

The water delivery system comprises a fluid passage for delivering heated water to the water delivery head 40, which in turn delivers the heated water to the brew chamber 18. The water delivery head comprises a water distribution disc. The water distribution disc provides an area of water delivery to the ground coffee. The water delivery head may further comprise a filter that enables the passage of water while also retaining the ground coffee so that it may be compacted during tamping.

A closing and tamping system is provided for compacting the ground coffee in the brew chamber 18 by providing relative movement between the water delivery head 40 and the brew chamber. This relative displacement furthermore applies force to the ground coffee to perform tamping.

The tamping system includes a hydraulic actuator 42, comprising a piston which is driven by hydraulic pressure. The hydraulic actuator may further comprise a return spring (not shown), to help retracting the piston after brewing.

A water outlet from the water pump 36 is coupled to the hydraulic actuator 42 by a first fluid coupling 44. The water outlet from the water pump is also coupled to the water delivery head 40 by a second fluid coupling 46. The second fluid coupling includes a passive in-line valve 48. The passive in-line valve opens when the pressure at the inlet side of the hydraulic actuator (i.e. at branch point 50A) reaches a desired pressure, e.g. a desired tamping pressure.

Below this pressure, the valve 48 stays closed. There may be hysteresis, so that the valve 48 opens at a first threshold pressure (an opening pressure), but only recloses when a lower second threshold pressure (a closing pressure) is reached. Alternatively, there may be only one threshold pressure.

In this way, the water pump 36 is used for both water delivery for brewing as well as water delivery for closing and tamping. The passive in-line valve 48 switches automatically between these two water delivery functions, without the need for user interaction or electrical actuators. The passive valve 48 opens for instance when a tamping pressure has been reached, i.e. when tamping has been completed. Water delivery then takes place to the water delivery head via the open valve 48. During this water delivery to the water delivery head, the tamping pressure is maintained.

This only one example of the components and connection of a fully automatic coffee machine. The controller 38 controls the brewing parameters as well as the movement of the brew chamber.

Fig. 2 also shows a bean reservoir 54 and a coffee grinder 56 having a ground coffee outlet 58 which delivers ground coffee to the brew chamber 58.

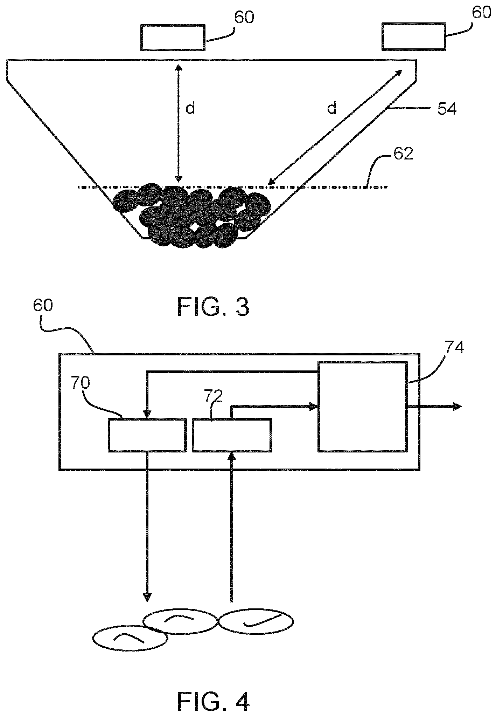

Fig. 3 shows a system 60 for analyzing coffee beans, in particular in a coffee bean reservoir 54. It may be incorporated into the coffee machine of Figure 1 or Figure 2.

The system is for sensing a level of darkness of the coffee beans and also measures a distance d to the coffee beans, in particular to the level 62 of the top of the coffee beans. Fig. 3 shows two possible positions for the system 60. The system is mounted on top of the bean reservoir 54, with a light input/output directed at the bean level.

The system may be positioned centrally over the reservoir or it may positioned over a periphery of the top opening of the reservoir and these two positions are shown in Figure 3). Thus, the system may be vertically above the beans or at an angle. The reservoir may have a glass lid over which the system is mounted or it may have an open top.

The distance measurement is preferably based on a time of flight measurement.

Fig. 4 shows the system components. The system comprises an infrared (IR) laser emitter 70 and a receiver 72 capable of detecting a returned infrared pulse. A processor 74 times the generation of an IR pulse and the return of a reflected pulse, and analyzes the light strength of the reflected pulse. The distance d between the beans and the sensor is measured, based on the travel time of the IR laser pulse.

The reflectance of the beans can then be calculated, based on the distance and the return intensity of the IR pulse.

This is possible using only an IR pulse because a dark coffee bean (i.e. one which absorbs visible light) will also absorb IR light as a function of the level of darkness.

The processor 74 may then connect to the main controller 38 of a coffee machine to enable automated control of the coffee machine in dependence on the roast intensity of the coffee beans.

In this way, there is a light sensor (IR in this example) for sensing a level of darkness of a coffee bean in the line of sight of the IR pulse, and a distance sensor for determining a distance between the light sensor and the coffee bean. The processor calibrates the sensed level of darkness using the determined distance, thereby to obtain a calibrated level of darkness. The calibrated level of darkness then represents a roasting intensity of the coffee beans.

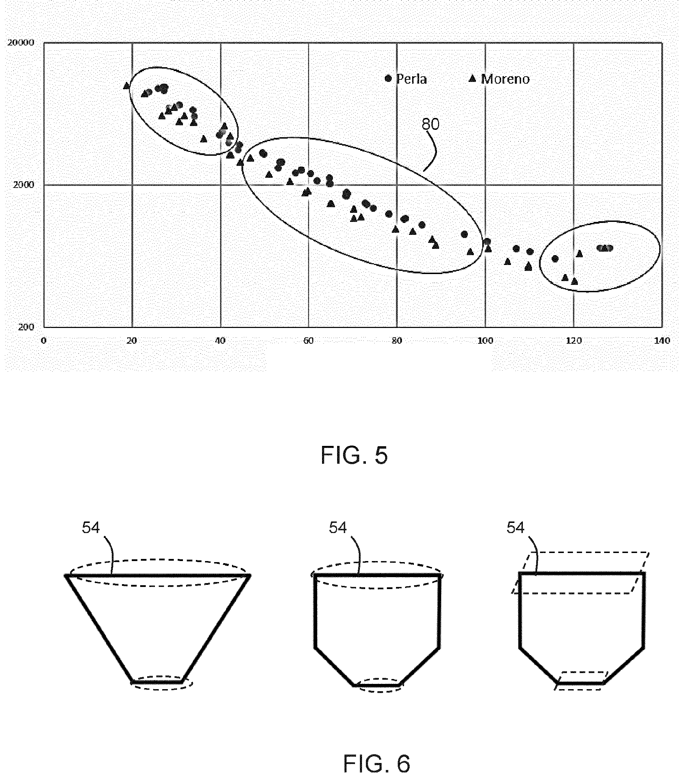

Fig. 5 is used to show how the calibration enables more accurate darkness determination. It shows results of a test with two bean types and roasting intensity; Perla (medium roast) and Moreno (dark roast).

Fig. 5 shows as the y-axis the reflected intensity (from a fixed intensity emitted IR pulse) in arbitrary units with a logarithmic scale, and as the x-axis the distance in mm between the sensor and the coffee bean along the line of sight of the sensor.

The graph shows a clear near linear relationship between the measured distance and the measured reflecting intensity value for both types of beans. The Perla (medium roast) beans produce higher reflectance intensity compared to the Moreno (dark roast) beans.

The zone 80 indicates the area were roast intensity measurements can reliably distinguish between bean types (for the particular sensor used).

The system thus measures a level of darkness of a coffee bean, rather than a color, and this enables simpler sensing hardware. In particular, in this example, the same IR pulse emission and detection is used both for time of flight determination and darkness level sensing. The calibration with distance makes the darkness level measurement more accurate.

The system has pre-stored relationships of the type shown in Fig. 5, so that a measured result (which will be a single point in the plot) can be paired with the closest pre-stored function, and thereby the roast level can be determined. The roast level is for example defined as one of three or more categories, such as three or five categories. The relationship between roast color and roast level is relatively independent of bean type so that the (calibrated) darkness level is a good indicator of roast level for all bean types.

When incorporated into a coffee machine, the system enables the main controller 38 of the coffee machine to take various actions. For example, the coffee machine may inform the user on the type of beans that have been placed in the bean reservoir, or choose the right beans if multiple bean reservoirs are present in the coffee machine.

Brew parameters may then be adjusted automatically to optimal settings, such as a grinder fineness/coarseness setting, a dosing amount from the grinder to the coffee vessel, and other brew parameters.

The coffee machine may also recommend recipes which are particularly suitable for the particular bean roast level, and similarly recommend a milk type in the case of milk-based coffee recipes.

The analysis system is described above as enabling a roast level to be determined. In addition, the same sensing system may be used to determine a quantity of coffee beans in the coffee bean reservoir based on the determined distance. The distance can be assumed to be a top level of the coffee beans, and hence the fill status of the coffee bean reservoir is determined. This additional information may be used in various ways by the coffee machine. For example, the controller may be adapted to control, in dependence on the determined quantity of coffee beans, the coffee grinding (grinding time or grinding setting). For example the system may prevent a grinding operation when the system is almost out of beans or it may enable bean level indications or warnings to be given to the consumer.

It can also be used for automatic bean ordering, and for ensuring the coffee machine will not run out of beans during coffee brewing. This may be achieved by a coffee machine which has internet connectivity.

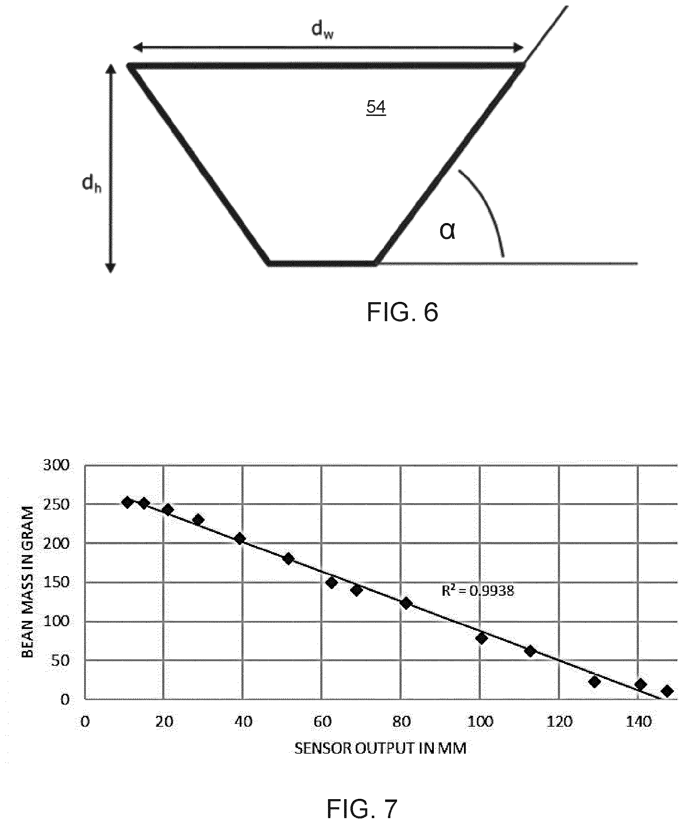

The invention can be applied to any bean reservoir shape and simply requires a mapping of bean level in the reservoir to the volume occupied. Fig. 6 shows three examples of coffee bean reservoir 54, namely a truncated cone shaped reservoir (left image), a circular cylinder shaped reservoir with a cone shaped funnel at the bottom, to allow flow of beans (middle image), and a square cylinder shaped reservoir with an angled bottom to allow flow of beans (right image).

The coffee bean reservoirs for example hold a maximum mass of coffee beans in the range 150g to 400g.

The wall angle α shown in Fig. 6 is for example from 25° to 90° (vertical).

The width dw is typically from 100mm to 200mm. A too small width will cause issues with the sensor placement. The maximum width is for example determined by the range of the sensor used and the minimum required wall angle.

The height dh is typically from 100mm to 200mm. The minimum height is determined by the minimum required wall angle and the maximum height is determined by the range of the used sensor.

Fig. 7 shows the results of a test, and shows the bean mass (y-axis in grams) versus the sensor output distance (x-axis in mm) for a 500ml beaker. The graph shows a linear relation between distance and the mass of beans.

The output of the sensor can be used to indicate on a user interface how many beans are in the reservoir. If the coffee machine is connected to the internet, an app may instead be used to indicate how many beans are in the reservoir. The coffee machine may then automatically order beans at a certain pre-set bean level or at a level indicated by the consumer, and/or the consumer can be warned to fill the reservoir or order new beans.

Knowledge of the amount of coffee beans may also be used to prevent the coffee machine from making a certain recipe when the bean level is too low. The system may also detect when new beans have been filled and ask the user if settings should be adjusted based on the new beans.

The example above is based on the use of an IR pulse for both reflection measurement and time of flight measurement. However, the distance measurement and the darkness estimate may be separate systems. The distance measurement may for example use a different part of the electromagnetic spectrum to the darkness measurement. A shared sensor could still be used by having filters for distinguishing between distance measurement light and darkness sensing light.

In the examples above, the distance measurement is based on an optical time of flight sensor. However, the distance may be determined in other ways, as outlined below:

### Capacitive measurement

This may involve the use of an electrode arrangement on the container wall. The presence of beans near an electrode will increase the relative permeability, and therefore, increase the total measured capacitance. Different electrode arrangements may be used, including a single electrode, a single electrode with an additional reference electrode to compensate for the bean characteristics, and multiple electrodes for discrete level sensing. The capacitance measurement may use comb electrodes forming an interdigital sensor.

### Ultrasonic sensing

An ultrasonic distance sensor can be used to measure a distance between the bean level and the sensor. The sensor uses a sound emitter and a receiver. The emitter sends a sound pulse through air. This pulse will reflect from the nearest object and return the pulse back to the sound receiver. The distance between the sensor and the object can thus be based on an acoustic time of flight.

### Light reflection

The presence of beans can be used to block a reflection path from a reflector strip so that a bean level can be determined as the first height at which the reflection path is present. Light gates may be provided at multiple points to provide a level sensing function.

### IR proximity sensor based on reflection

A sensor may comprise an IR light emitter and a photodiode. The emitter is used to send IR pulses and when there is an object present, this light will be reflected by the object back to a photodiode. The beans reflect light so that the sensor can be used to detect the bean presence at a single point. Multiple levels can be detected by using multiple sensors.

The invention is of particular interest for a fully automatic coffee machine, but it may be applied to any type of coffee machine incorporating an internal coffee bean reservoir (and grinder). It may also be applied to a standalone grinder for controlling the grinding settings (e.g. grind size and grinding duration).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If the term "adapted to" is used in the claims or description, it is noted the term "adapted to" is intended to be equivalent to the term "configured to". Any reference signs in the claims should not be construed as limiting the scope.

## Claims

1. A system for analyzing coffee beans, comprising:
a light sensor (72) for sensing a level of darkness of a coffee bean;
a distance sensor (72,74) for determining a distance between the light sensor and the coffee bean; and
a processor (74) adapted to calibrate the sensed level of darkness using the determined distance, thereby to obtain a calibrated level of darkness.

2. The system of claim 1, wherein the light sensor is for measuring a reflectance from the coffee bean.

3. The system of claim 1 or 2, wherein the distance sensor comprises a time of flight sensor.

4. The system of claim 3, wherein the time of flight sensor comprises a light source, and wherein the time of flight sensor uses the light sensor to determine a time of flight.

5. The system of claim 4, wherein the light source comprises an infrared laser and the light sensor is adapted to sense infrared light.

6. The system of any one of claims 1 to 5, wherein the processor is adapted to output a roast level from the calibrated level of darkness.

7. A coffee machine, comprising:
a water reservoir (30);
a water heater (34);
a water pump (36);
a coffee bean reservoir (54);
a coffee grinder (56) having a ground coffee outlet;
a coffee vessel (18) for receiving ground coffee from the coffee grinder;
a water delivery system for delivering heated water to the coffee vessel;
the analyzing system of claim 6 for determining a roast level of coffee beans in the coffee bean reservoir; and
a controller (38) for controlling coffee brewing.

8. The coffee machine of claim 7, wherein the controller is adapted to control, in dependence on the determined roast level, at least one of:
coffee brewing parameters;
coffee grinding parameters;
the dosing of ground coffee to the coffee vessel.

9. The coffee machine of claim 7 or 8, wherein the controller is adapted to provide an output to a user indicating one or more of:
the determined roast level;
coffee recipe advice for the determined roast level;
coffee taste options for the determined roast level;
a detected change in the coffee bean type.

10. The coffee machine of any one of claims claim 7 to 9, wherein the processor of the analyzing system is further adapted to determine a quantity of coffee beans in the coffee bean reservoir based on the determined distance.

11. The coffee machine of claim 10, wherein the controller is adapted to control, in dependence on the determined quantity of coffee beans, the coffee grinding.

12. The coffee machine of claim 10 or 11, wherein the controller is adapted to determine, in dependence on the determined quantity of coffee beans, which coffee recipes can be completed.

13. The coffee machine of any one of claims 10 to 12, wherein the controller is adapted to implement automatic bean ordering, in dependence on the determined quantity of coffee.

14. The coffee machine of any one of claims 10 to 13, wherein the controller is adapted to provide an output signal indicating the remaining quantity of coffee beans.

15. A coffee grinding system, comprising:
a coffee bean reservoir for coffee beans;
a coffee grinder;
the analyzing system of claim 6 for determining a roast level of coffee beans in the coffee bean reservoir; and
a controller (38) for controlling coffee the coffee grinder settings based on the roast level.
